# EUROPEAN PATENT APPLICATION

(11) **EP 3 730 108 A1**
(43) Date of publication of application: **28.10.2020**
(21) Application number: 20169390.0
(22) Date of filing: 14.04.2020
(51) Int. Cl.: A61F 13/47, A61F 13/471, A61F 13/472, A61F 13/551, A61F 13/56

(54) **URINE ABSORBING PAD PACKAGE**

(30) Priority: 26.04.2019 JP 2019085895
(71) Applicant: Unicharm Corporation, Shikokuchuo-shi, Ehime 799-0111 (JP)
(72) Inventor: TADA, Mikihiko, Kanonji-shi, Kagawa 769-1602 (JP); KOSAKA, Shoshi, Kanonji-shi, Kagawa 769-1602 (JP)
(74) Representative: Staeger & Sperling Partnerschaftsgesellschaft mbB

(57) **Abstract**

A urine absorbing pad package (1) includes a urine absorbing pad (10), a packaging sheet(80), and a release sheet (70).In an unfolded state in which the urine absorbing pad is not folded with the width folding line (FW) as a base point, a sheet extension region (81) in which the packaging sheet extends outside a sheet joining portion (76) in the front-rear direction is provided on one end side (1X) of the urine absorbing pad package in the front-rear direction. In the unfolded state, a pad extension region (18) in which the urine absorbing pad extends outside the packaging sheet in the front-rear direction on another end side (1Y) of the urine absorbing pad package in the front-rear direction. In the one end side of the urine absorbing pad package, at least a portion of the side adhesive portion (22) is disposed outside the contraction portion (13) in the front-rear direction and outside the center adhesive portion (21) in the front-rear direction.

## Description

### [TECHNICAL FIELD]

The present invention relates to a urine absorbing pad package including a urine absorbing pad and a packaging sheet for packaging the urine absorbing pad.

### [BACKGROUND ART]

Patent Literature 1 discloses a urine absorbing pad package which includes a urine absorbing pad which has a front-rear direction and a width direction orthogonal to each other, a packaging sheet which is disposed on a non-skin surface side of the urine absorbing pad, and a release sheet which is disposed between the urine absorbing pad and the packaging sheet. The release sheet covers an adhesive portion for joining the urine absorbing pad to a wearing article and is joined to the packaging sheet via a sheet joining portion. As illustrated in Fig. 4 of Patent Literature 1, in the package, the urine absorbing pad is individually packaged by the packaging sheet in a folded state in which the package is folded with a folding line at least along the width direction as a base point.

When a user uses the urine absorbing pad, the user unfolds the urine absorbing pad from the individually packaged state to an unfolded state and removes the urine absorbing pad from the packaging sheet and the release sheet. In this case, generally, the user removes the urine absorbing pad from the packaging sheet while unfolding the packaging sheet and the urine absorbing pad in a state of holding the packaging sheet with one hand and the urine absorbing pad with the other hand. According to this operation, it is possible to perform an operation of unfolding the package and an operation of removing the urine absorbing pad by one operation.

### [CITATION LIST]

### [PATENT LITERATURE]

Patent Literature 1: JP 2019-42070 A

### [SUMMARY OF INVENTION]

In addition, the urine absorbing pad is folded along the width direction with the folding line as the base point before use. Accordingly, a crease is formed in the urine absorbing pad. When the package is unfolded in a state where each of the packaging sheet and the urine absorbing pad is held, in a region in which the urine absorbing pad is directly held with one hand, a force is easily applied to the urine absorbing pad and the urine absorbing pad is easily unfolded to face the crease. However, in a region in which the packaging sheet is held with the other hand, a force cannot be directly applied to the urine absorbing pad and the force is applied to the urine absorbing pad via the packaging sheet. Therefore, it is not possible to unfold the urine absorbing pad in a flat state facing the crease.

In addition, generally, the user generally holds the vicinity of a center of the package in a width direction thereof in order to unfold the package over the entire region. Therefore, in particular, the urine absorbing pad cannot be unfolded to a sufficiently flat state in a side portion of the urine absorbing pad in the width direction and the urine absorbing pad may be attached to the wearing article in a state where the side portion of the urine absorbing pad in the width direction is folded inward. As a result, there are inconveniences in that a body cannot be appropriately covered by the urine absorbing pad, which causes leakage of the urine, and the urine absorbing pad is worn in a state where the urine absorbing pad is folded inside, and thus, a wearing feeling deteriorates.

Therefore, there is a demand for a urine absorbing pad package capable of preventing the side portion of the urine absorbing pad in the width direction from being folded inward during attachment and preventing occurrence of the leakage and the deterioration of the wearing feeling.

A urine absorbing pad package of one embodiment comprising: a urine absorbing pad that has a front-rear direction and a width direction orthogonal to each other; a packaging sheet disposed on a non-skin surface side of the urine absorbing pad; and a release sheet disposed between the urine absorbing pad and the packaging sheet,
at least the urine absorbing pad being folded with a width folding line as a base point along the width direction, the urine absorbing pad being individually packaged by the packaging sheet. The urine absorbing pad includes an absorbent core, a center sheet that covers the absorbent core on a skin surface side of the absorbent core, an upright leakage-proof gather that is disposed on a skin surface side of the center sheet and has a contraction portion contracted in the front-rear direction, a backsheet disposed on a non-skin surface side of the absorbent core, and an adhesive portion that is disposed on a non-skin surface side of the backsheet and joined to a wearing article when the urine absorbing pad is used. The adhesive portion includes a center adhesive portion disposed at a center of the urine absorbing pad in the width direction and a side adhesive portion disposed outside of the center adhesive portion in the width direction. The release sheet covers the side adhesive portion before the urine absorbing pad is used. The packaging sheet is joined to the release sheet via a sheet joining portion. In an unfolded state in which the urine absorbing pad is not folded with the width folding line as a base point, a sheet extension region in which the packaging sheet extends outside the sheet joining portion in the front-rear direction is provided on one end side of the urine absorbing pad package in the front-rear direction. In the unfolded state, a pad extension region in which the urine absorbing pad extends outside the packaging sheet in the front-rear direction on another end side of the urine absorbing pad package in the front-rear direction. In the one end side of the urine absorbing pad package, at least a portion of the side adhesive portion is disposed outside the contraction portion in the front-rear direction and outside the center adhesive portion in the front-rear direction.

### [BRIEF DESCRIPTION OF DRAWINGS]

Fig. 1 is a developed plan view when a urine absorbing pad package according to an embodiment is viewed from a skin surface side.
Fig. 2 is a developed plan view when the urine absorbing pad package according to the embodiment is viewed from a non-skin surface side.
Fig. 3 is a developed plan view when the urine absorbing pad according to the embodiment is viewed from the non-skin surface side.
Fig. 4 is a cross-sectional view taken along line A-A illustrated in Fig. 1.
Fig. 5 is a cross-sectional view taken along line B-B illustrated in Fig. 1.
Fig. 6 is a view for explaining a state where the urine absorbing pad package according to the embodiment is folded.
Fig. 7 is a view for explaining a state in which the urine absorbing pad of the urine absorbing pad package according to the embodiment is removed.

### [DESCRIPTION OF EMBODIMENTS]

### (1) Outline of Embodiments

From the present specification with reference to the accompanying drawings, at least the following matters will be disclosed. A urine absorbing pad package of one embodiment comprising: a urine absorbing pad that has a front-rear direction and a width direction orthogonal to each other; a packaging sheet disposed on a non-skin surface side of the urine absorbing pad; and a release sheet disposed between the urine absorbing pad and the packaging sheet, at least the urine absorbing pad being folded with a width folding line as a base point along the width direction, the urine absorbing pad being individually packaged by the packaging sheet. The urine absorbing pad includes an absorbent core, a center sheet that covers the absorbent core on a skin surface side of the absorbent core, an upright leakage-proof gather that is disposed on a skin surface side of the center sheet and has a contraction portion contracted in the front-rear direction, a backsheet disposed on a non-skin surface side of the absorbent core, and an adhesive portion that is disposed on a non-skin surface side of the backsheet and joined to a wearing article when the urine absorbing pad is used. The adhesive portion includes a center adhesive portion disposed at a center of the urine absorbing pad in the width direction and a side adhesive portion disposed outside of the center adhesive portion in the width direction. The release sheet covers the side adhesive portion before the urine absorbing pad is used. The packaging sheet is joined to the release sheet via a sheet joining portion. In an unfolded state in which the urine absorbing pad is not folded with the width folding line as a base point, a sheet extension region in which the packaging sheet extends outside the sheet joining portion in the front-rear direction is provided on one end side of the urine absorbing pad package in the front-rear direction. In the unfolded state, a pad extension region in which the urine absorbing pad extends outside the packaging sheet in the front-rear direction on another end side of the urine absorbing pad package in the front-rear direction. In the one end side of the urine absorbing pad package, at least a portion of the side adhesive portion is disposed outside the contraction portion in the front-rear direction and outside the center adhesive portion in the front-rear direction.

According to this aspect, it is possible to suppress an inconvenience that the urine absorbing pad is peeled off from the wearing article due to the side portion of the urine absorbing pad in the width direction being folded inward during attachment, and it is possible to prevent deterioration of a wearing feeling due to the urine absorbing pad being folded inward during wearing.

According to a preferred aspect, at least a portion of the sheet extension region may extend outside the urine absorbing pad in the front-rear direction in the unfolded state. According to this aspect, the user can easily hold the sheet extension region extending from the urine absorbing pad. In the one end of the package, a user can easily hold only the packaging sheet without holding both the packaging sheet and the urine absorbing pad and can more easily perform an operation of unfolding the package and an operation of removing the urine absorbing pad by one operation.

According to a preferred aspect, an outer end portion of the pad extension region in the front-rear direction may have a folded region folded toward the non-skin surface side in a state of being folded with the width folding line as a base point. According to this aspect, the folded region of the pad extension region easily stands on the non-skin surface side due to the crease. Therefore, when the urine absorbing pad is removed, the user can hold the folded region located at the outer end portion of the pad extension region in the front-rear direction. The region held by the user can be provided at the outer end portion of the urine absorbing pad in the front-rear direction, and the entire urine absorbing pad can be more easily unfolded in the front-rear direction.

According to a preferred aspect, in the one end side of the urine absorbing pad package, at least a portion of the side adhesive portion may be disposed outside the absorbent core in the front-rear direction. According to this aspect, the urine absorbing pad is hardly bent due to rigidity of the side adhesive portion, and the urine absorbing pad is joined to the wearing article via the side adhesive portion, and thus, the urine absorbing pad is not easily bent.

According to a preferred aspect, in the one end side of the urine absorbing pad package, at least a portion of the sheet joining portion may be disposed outside the contraction portion in the front-rear direction. According to this aspect, in the one end side of the urine absorbing pad package, the sheet joining portion is located outside the contraction portion in the front-rear direction. Therefore, the urine absorbing pad can be pulled via the release sheet in a region where the contraction of the contraction portion is hardly affected. Therefore, the urine absorbing pad can be easily attached on the wearing article in a state of being unfolded to the vicinity of the side portion of the urine absorbing pad in the width direction.

According to a preferred aspect, in the one end side of the urine absorbing pad package, at least a portion of the sheet joining portion may be disposed outside the side adhesive portion in the front-rear direction. According to this aspect, since the sheet joining portion is located outside the side adhesive portion in the front-rear direction, when the user pulls the packaging sheet outward in the front-rear direction, a force is easily applied to the urine absorbing pad. Accordingly, the urine absorbing pad is more easily pulled outward in the front-rear direction.

According to a preferred aspect, in the one end side of the urine absorbing pad package, at least a portion of the sheet joining portion may be deviated outward in the front-rear direction with respect to a center of the packaging sheet in the front-rear direction. According to this aspect, the release sheet can be pulled via the packaging sheet outside the center of the packaging sheet in the front-rear direction. In one end portion of the urine absorbing pad in the front-rear direction, the urine absorbing pad can be unfolded to be flat, and the urine absorbing pad is easily attached to the wearing article in a state where the urine absorbing pad is flatter.

According to a preferred aspect, in one end side of the urine absorbing pad package, a distance between the side adhesive portion and an outer end edge of the urine absorbing pad in the front-rear direction may be shorter than a distance between the side adhesive portion and an outer edge of the urine absorbing pad in the width direction. According to this aspect, since the distance between the side adhesive portion and the outer end edge of the urine absorbing pad in the front-rear direction is relatively short, a flat state is easily maintained to the outer end edge of the urine absorbing pad during unfolding. Therefore, it is possible to suppress an inconvenience that the urine absorbing pad is folded inward at the outer end edge of the urine absorbing pad.

According to a preferred aspect, a dimension of the urine absorbing pad in the width direction may gradually decrease from the one end side of the urine absorbing pad package toward the other end side of the urine absorbing pad package. According to this aspect, in the one end side of the urine absorbing pad package, the urine absorbing pad is easily unfolded to the vicinity of the side portion of the urine absorbing pad in the width direction and even in a configuration in which a length of the one end side of the urine absorbing pad package in width direction is long, it is possible to suppress an inconvenience caused by the side portion of the urine absorbing pad in the width direction being folded inward.

According to a preferred aspect, at least a portion of the contraction portion may be disposed outside the absorbent core in the width direction. According to this aspect, in the one end side of the urine absorbing pad package, the urine absorbing pad is easily unfolded to the vicinity of the side portion of the urine absorbing pad in the width direction, and even in a configuration in which the contraction portion is disposed outside the absorbent core in the width direction, it is possible to suppress the inconvenience caused by the side portion of the urine absorbing pad in the width direction being folded inward.

According to a preferred aspect, the side adhesive portion may be disposed in at least one of a region overlapping the contraction portion and a region extending from the contraction portion in the front-rear direction. According to this aspect, since the side adhesive portion is disposed in at least one of the region overlapping the contraction portion and the region extending from the contraction portion in the front-rear direction, the urine absorbing pad is joined to the wearing article via the side adhesive portion during wearing. Accordingly, the side portion of the urine absorbing pad in the width direction is hardly bent inward.

### (2) Embodiment of Urine Absorbing Pad

Hereinafter, a urine absorbing pad package according to an embodiment will be described with reference to the drawings. Moreover, in the following descriptions of the drawings, the same or similar portions are denoted by the same or similar reference numerals. However, it should be noted that the drawings are schematic and ratios of dimensions are different from actual dimensions. Therefore, specific dimensions and the like should be determined in consideration of the following description. Moreover, the drawings may include portions having different dimensional relationships and ratios.

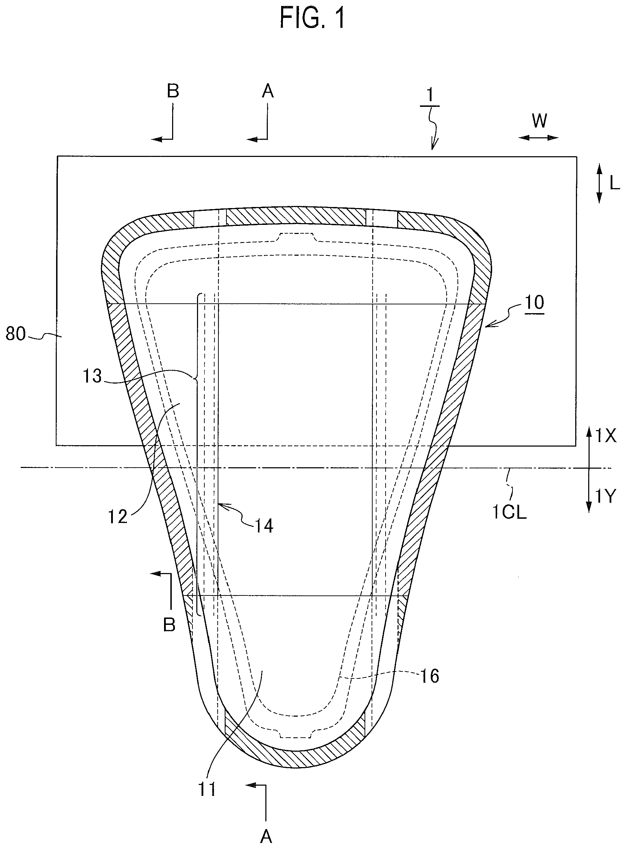

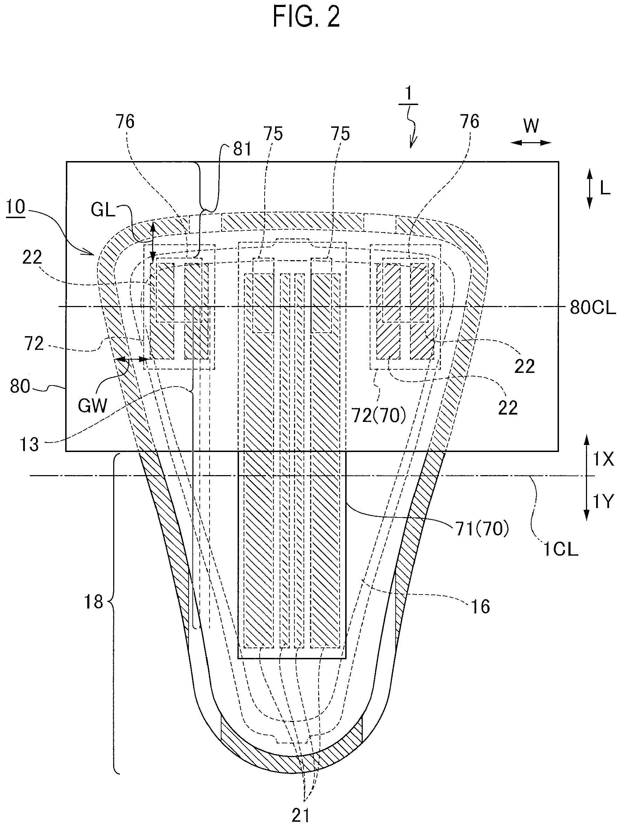

Fig. 1 is a developed plan view when a urine absorbing pad package 1 according to the present embodiment is viewed from a skin surface side T1, and Fig. 2 is a developed plan view when the urine absorbing pad package 1 according to the embodiment is viewed from a non-skin surface side T2.

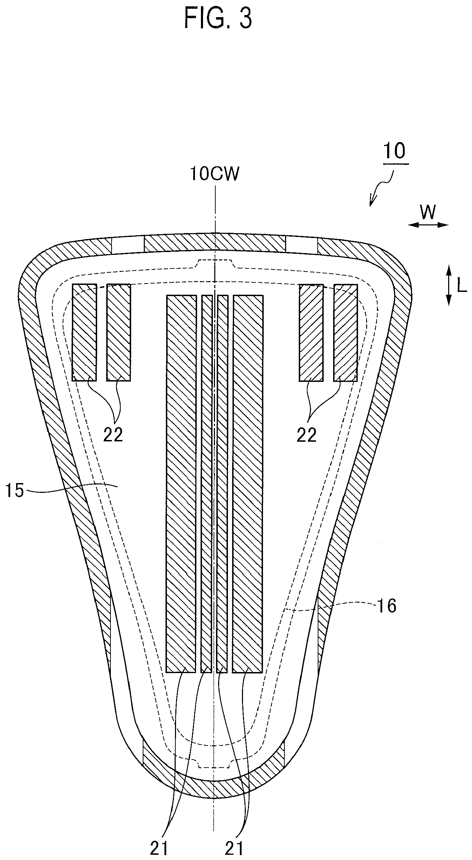

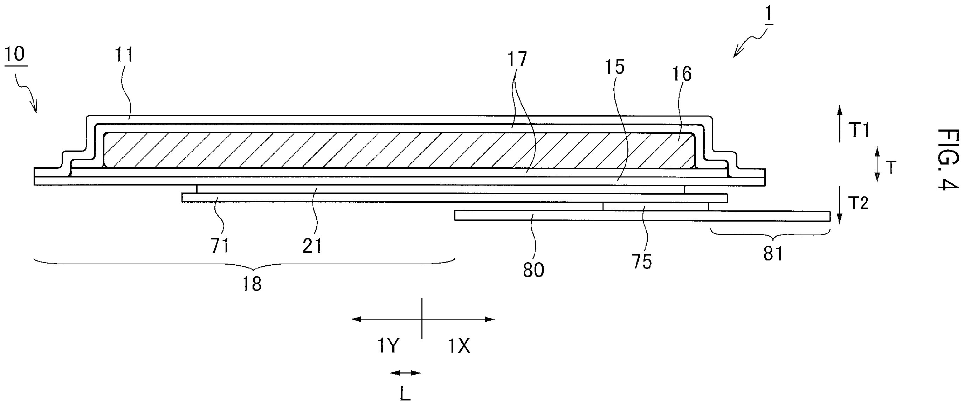

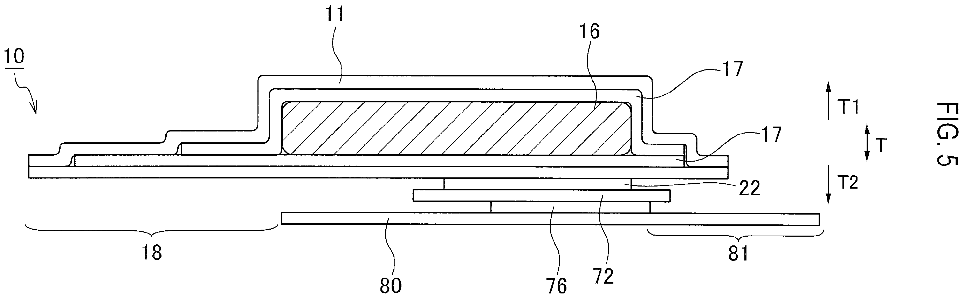

Fig. 3 is a developed plan view when the urine absorbing pad is viewed from the non-skin surface side T2. The developed plan views illustrated in Figs. 1 to 3 illustrate an unfolded state in which the urine absorbing pad is unfolded (not folded with a folding line as a base point). Fig. 4 is a cross-sectional view taken along line A-A illustrated in Fig. 1 and Fig. 5 is a cross-sectional view taken along line B-B illustrated in Fig. 1. The urine absorbing pad 10 of the present embodiment is a male urine absorbing pad and is used in a state of being attached inside a wearing article such as underwear. In addition, the urine absorbing pad of the present invention is not limited to the male urine absorbing pad but may be a female urine absorbing pad. The following drawings illustrate an extension state in which a leakage-proof gather of the urine absorbing pad extends to a state in which no wrinkle is formed. Further, in the following description, positional relationships and states of constituent members are positional relationships and states in the extension state unless otherwise specified.

The urine absorbing pad package 1 includes the urine absorbing pad 10, a packaging sheet 80, and a release sheet 70. The urine absorbing pad 10 has a front-rear direction L and a width direction W orthogonal to each other. The front-rear direction L is defined by a direction extending to a front side of a body and a rear side of the body. In other words, the front-rear direction L is a direction extending forth and back in the unfolded urine absorbing pad 10. In addition, the urine absorbing pad 10 has a thickness direction T orthogonal to both the front-rear direction L and the width direction W. The thickness direction T extends to the skin surface side T1 facing a wearer side and the non-skin surface side T2 away from the wearer. In the present specification, the "skin surface side" corresponds to a side facing a skin of the wearer during use. The "non-skin surface side" corresponds to a side facing a side opposite to the skin of the wearer during use.

The urine absorbing pad 10 is attached to a wearing article such as underwear during use. More specifically, the urine absorbing pad 10 is disposed forward from a crotch of the wearer so as to be deviated to a front side of a center of the wearing article in the front-rear direction. The urine absorbing pad 10 may be attached so as to cover a male urination outlet. A dimension of the urine absorbing pad 10 in the width direction W gradually decreases from the front side to the rear side of the urine absorbing pad and may be symmetric with respect to a center line of the urine absorbing pad in the front-rear direction. The dimension of a front end portion of the urine absorbing pad 10 in the width direction W may be longer than a dimension of a rear end portion of the urine absorbing pad 10 in the width direction W. Moreover, in the urine absorbing pad according to a modification example, a dimension of the urine absorbing pad in the width direction W does not gradually decrease from a front side to a rear side of the urine absorbing pad and may be symmetric with respect to a center line of the urine absorbing pad in a front-rear direction. Moreover, the front end portion and the rear end portion in the present invention are portions occupying a certain range in the front-rear direction L including an edge in the front-rear direction L, and a front end edge and a rear end edge are edges in the front-rear direction L. An outer end portion includes the front end portion and the rear end portion, and an outer end edge includes the front end edge and the rear end edge.

The urine absorbing pad 10 may include an absorbent core 16, a center sheet 11, a leakage-proof gather 14, a backsheet 15, and an adhesive portion. For example, the absorbent core 16 may be a laminate of absorbent material including ground pulp, a superabsorbent polymer (SAP), or a mixture thereof. For example, the absorbent core 16 may be covered with a core wrap 17 made of a tissue or a nonwoven sheet.

The center sheet 11 covers the absorbent core 16 on the skin surface side T1 of the absorbent core 16. The center sheet 11 only needs to cover at least a center of the absorbent core 16 in the width direction. The center sheet 11 may be a liquid-permeable sheet. The urine absorbing pad 10 may have a side sheet 12 which covers a side of the center sheet 11 on the skin surface side T1 of the absorbent core 16. An inner portion of the side sheet 12 is folded with the folding line as a base point along the front-rear direction. A leakage-proof elastic member extending in the front-rear direction may be disposed between the folded side sheets 12. The side sheet 12 and the leakage-proof elastic member constitute an upright leakage-proof gather 14. The leakage-proof gather 14 is disposed on the skin surface side T1 of the center sheet 11, and a contraction portion 13 which is contracted in the front-rear direction L. The contraction portion 13 is a portion which is fixed to the side sheet 12 in a state where the leakage-proof elastic member extends, in other words, the contraction portion 13 is an effective length portion of the leakage-proof elastic member.

The backsheet 15 is disposed on the non-skin surface side T2 of the absorbent core 16. The backsheet 15 may be a liquid-impermeable sheet. The adhesive portion which is joined to the wearing article when the urine absorbing pad 10 is used may be disposed on the surface of the backsheet 15 on the non-skin surface side T2. Fig. 3 is a plan view when the package 1 in a state where the packaging sheet 80 and the release sheet 70 are moved, that is, only the urine absorbing pad 10 is viewed from the non-skin surface side T2. The adhesive portion may be disposed on the non-skin surface side T2 of the backsheet 15. The adhesive portion has a center adhesive portion 21 which is disposed at the center of the urine absorbing pad 10 in the width direction W and a side adhesive portion 22 which is disposed outside the center adhesive portion 21 in the width direction W. The center adhesive portion 21 only needs to be disposed in the vicinity of the center of the urine absorbing pad 10 in the width direction W, and may straddle a center 10CW of the urine absorbing pad 10 in the width direction, or may be disposed so as not to overlap the center 10CW of the urine absorbing pad in the width direction. A plurality of the center adhesive portion 21 may extend in the front-rear direction L and may be disposed across the center 10CW of the urine absorbing pad 10 in the width direction. A plurality of the side adhesive portion 22 may extend in the front-rear direction L and may be disposed at intervals in the width direction W. A length of the side adhesive portion 22 in the front-rear direction L is shorter than a length of the center adhesive portion 21 in the front-rear direction L. In one end side 1X of the package described later, at least a portion of the side adhesive portion 22 is disposed outside the contraction portion 13 of the leakage-proof gather 14 in the front-rear direction L and outside the center adhesive portion 21 in the front-rear direction L.

The release sheet 70 covers the adhesive portion to protect the adhesive portion before the urine absorbing pad 10 is used. The release sheet 70 may include a center release sheet 71 which covers the center adhesive portion 21 and a side release sheet 72 which covers the side adhesive portion 22. The "release sheet" of the present invention only needs to cover the side adhesive portion 22 and is constituted by the side release sheet 72 of the present embodiment. Moreover, the side release sheet 72 and the center release sheet 71 may be integrated with each other. In the present embodiment, the center release sheet 71 and the side release sheet 72 may be formed separately and may be disposed at intervals in the width direction W. The release sheet 70 is disposed between the packaging sheet 80 and the urine absorbing pad 10 in a region which overlaps the packaging sheet 80 in a plan view, and is located on the non-skin surface side T2 of the urine absorbing pad 10 in a region which does not overlap the packaging sheet 80 in a plan view and is exposed in the unfolded state.

The packaging sheet 80 may overlap at least a portion of the urine absorbing pad in a plan view. The packaging sheet 80 is disposed on the non-skin surface side T2 of the urine absorbing pad 10. The packaging sheet 80 is joined to the release sheet 70 via a sheet joining portion. More specifically, the packaging sheet 80 is joined to the center release sheet 71 via a center sheet joining portion 75 and is joined to the side release sheet 72 via a side sheet joining portion 76. The "sheet joining portion" of the present invention joins the side release sheet 72 which covers the side adhesive portion 22 and the packaging sheet 80 to each other and is constituted by the side sheet joining portion 76 of the present embodiment.

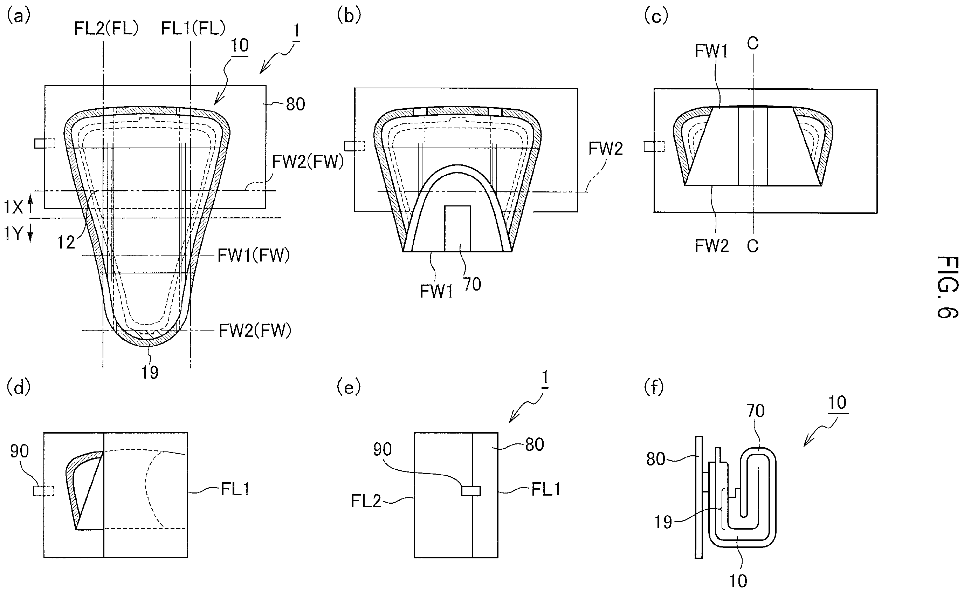

Next, a folded state and the unfolded state of the urine absorbing pad package 1 will be described with reference to Fig. 6. In the urine absorbing pad package 1 of the present embodiment, the urine absorbing pad 10, the release sheet 70, and the packaging sheet 80 are all folded with the width folding line FW and the front-rear folding line FL as base points. Moreover, in a package according to a modification example, at least the urine absorbing pad may be folded with the width folding line as a base point. The width folding line FW only needs to extend in the width direction W and may extend along the width direction W. The width folding line FW of the present embodiment has a first width folding line FW1 and a second width folding line FW2. The front-rear folding line FL only needs to extend at least in the front-rear direction L and may extend along the front-rear direction L. The front-rear folding line FL of the present embodiment has a first front-rear folding line FL1 and a second front-rear folding line FL2.

Fig. 6(a) illustrates the package in the unfolded state. In the urine absorbing pad package 1 in the unfolded state, the release sheet 70 and the urine absorbing pad 10 are placed on the packaging sheet 80. A length of the packaging sheet 80 in the front-rear direction L is shorter than a length of the urine absorbing pad 10 in the front-rear direction L. In the unfolded state, the urine absorbing pad package 1 has the one end side 1X which is located on one end side of the package in the front-rear direction of a center 1CL of the package 1 in the front-rear direction L and the other end side 1Y which is located on the other end side of the package in the front-rear direction of the center 1CL of the package 1 in the front-rear direction L. The one end side 1X of the present embodiment is located on a front side of the center 1CL of the package 1 in the front-rear direction L and the other end side 1Y is located on a rear side of the center 1CL of the package 1 in the front-rear direction L.

The package 1 is folded inward in the front-rear direction L from the unfolded state with the first width folding line FW1 as a base point. The first width folding line FW1 is a folding line for folding the urine absorbing pad 10 is folded so that the urine absorbing pad 10 faces the skin surface side T1 and is a valley folding line when the package is viewed from the skin surface side. The first width folding line FW1 is provided on the other end side 1Y of the package 1. Fig. 6(b) illustrates a first width folded state in which the first width folding line FW1 is used as a base point.

The package 1 is folded inward in the front-rear direction L from a first width folded state with the second width folding line FW2 as a base point. The second width folding line FW2 is a folding line to be folded so that a region on the one end side 1X of the first width folding line FW1 faces the skin surface side and a region on the other end side 1Y of the first width folding line FW1 faces the non-skin surface side. The second width folding line FW2 is provided on the one end side 1X of the package 1. Fig. 6(c) illustrates a second width folded state in which the folding is performed with the second width folding line FW2 as a base point. Fig. 6(f) is a cross-sectional view along line C-C illustrated in Fig. 6(c).

The package 1 is folded inward in the width direction W from the second width folded state with the first front-rear folding line FL1 as a base point. Fig. 6(d) illustrates a first front-rear folded state in which the folding is performed with the first front-rear folding line FL1 as a base point. The package 1 is folded inward in the width direction W from the first front-rear folded state with the second front-rear folding line FL2 as a base point. Fig. 6(e) illustrates a second front-rear folded state in which the folding is performed with the second front-rear folding line FL2 as a base point. The second front-rear folded state is an individually packaged state in which the urine absorbing pad 10 and the release sheet 70 are individually packaged by the packaging sheet 80. In the individually packaged state, one end of the packaging sheet in the width direction of the urine absorbing pad is joined to an outer surface of the packaging sheet 80 by a tape member 90.

When a user uses the urine absorbing pad 10, the user opens the package 1 through a reverse process of the folding process. Specifically, the user performs unfolding from the individually packaged state to the first front-rear folded state, performs unfolding from the first front-rear folded state to the second width folded state, performs unfolding from the second width folded state to the first width folded state, and performs unfolding from the first width folded state to the unfolded state. In the unfolded state, in the package 1, the crease is formed by the width folding line FW and the crease is formed by the front-rear folding line FL. Accordingly, the urine absorbing pad 10 cannot be unfolded in a state where the urine absorbing pad 10 is sufficiently flat. The package 1 of the present invention is configured so that the urine absorbing pad 10 can be easily unfolded to a flat state during attachment in the package having the crease.

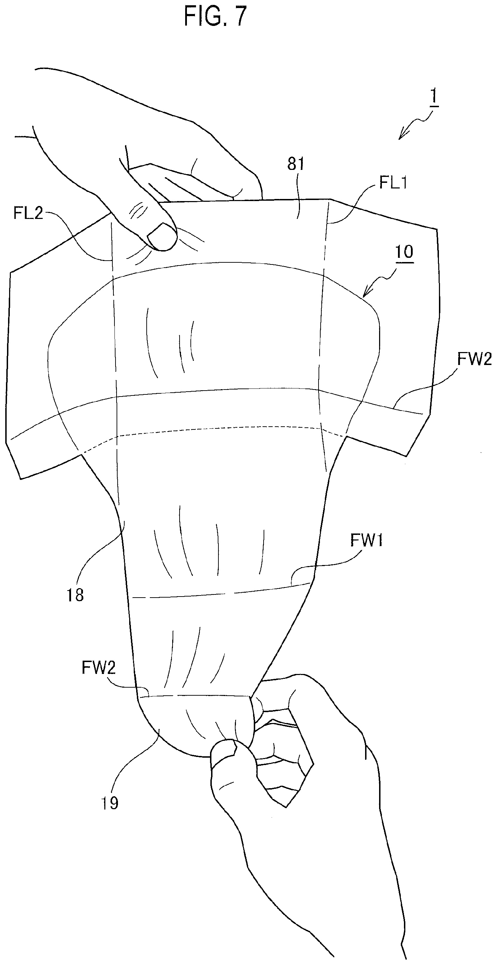

Next, a state where the urine absorbing pad 10 is removed during attachment will be described. When the user uses the urine absorbing pad, the user removes the urine absorbing pad from the packaging sheet while unfolding the package from the state individually packaged by the packaging sheet to the unfolded state. In this case, as illustrated in Fig. 7, in general, the user operates while holding the packaging sheet 80 with one hand and holding the urine absorbing pad 10 with the other hand. At this time, a sheet extension region 81 in which the packaging sheet 80 extends from the side sheet joining portion 76 is disposed on the one end side 1X of the package 1, and the user can hold the sheet extension region 81 with one hand. The sheet extension region 81 is a region of the packaging sheet 80 which extends outside the side sheet joining portion 76 in the front-rear direction L. Moreover, a pad extension region 18 in which the urine absorbing pad 10 extends from the packaging sheet is disposed on the other end side of the urine absorbing pad package 1, and the user can hold the pad extension region 18 with the other hand. The pad extension region 18 is a region of the urine absorbing pad 10 in the unfolded state which extends outside the packaging sheet in the front-rear direction. In addition, in general, the user holds the vicinity of the center of the package 1 in the width direction in order to unfold the entire package 1 in the width direction in the front-rear direction L.

The user does not directly hold the urine absorbing pad 10 on the one end side 1X of the urine absorbing pad package. Accordingly, the urine absorbing pad 10 is pulled to the unfolded state via the packaging sheet 80 and the release sheet 70. More specifically, the urine absorbing pad 10 is pulled through the adhesive portion joined to the release sheet 70. In this case, the side adhesive portion 22 is located outside the center adhesive portion 21 in the front-rear direction L. Accordingly, an outer portion (a portion where the side adhesive portion 22 is provided) of the urine absorbing pad 10 in the width direction W is pulled outward in the front-rear direction L of the center (a portion where the center adhesive portion 21 is provided) of the urine absorbing pad 10 in the width direction. Therefore, even in a case where the user holds the vicinity of the center of the urine absorbing pad 10 in the width direction to unfold the urine absorbing pad 10, the urine absorbing pad 10 can be unfolded in a flat state to the vicinity of the side portion of the urine absorbing pad 10 in the width direction W, and the urine absorbing pad 10 is not easily attached to the wearing article in a state where the side portion of the urine absorbing pad 10 in the width direction is folded inward.

In addition, the side adhesive portion 22 is located outside the contraction portion 13 of the leakage-proof gather 14 in the front-rear direction L on the one end side 1X of the urine absorbing pad package. Accordingly, the urine absorbing pad 10 can be pulled in a direction in which the urine absorbing pad 10 is unfolded via the side adhesive portion 22 in a region in which contraction of the contraction portion 13 is hardly affected. Therefore, the urine absorbing pad 10 can be easily attached to the wearing article in a state of being unfolded to the vicinity of the side portion of the urine absorbing pad 10 in the width direction W. Accordingly, it is possible to suppress an inconvenience that the urine absorbing pad 10 is peeled off from the wearing article due to the side portion of the urine absorbing pad 10 in the width direction W being folded inward during attachment, and it is possible to prevent deterioration of a wearing feeling due to the urine absorbing pad being folded inward during wearing.

At least a portion of the sheet extension region 81 may extend outside the urine absorbing pad 10 in the front-rear direction L in the unfolded state. More preferably, the sheet extension region 81 may be located outside an outer end edge of the urine absorbing pad 10 in the front-rear direction L. The user can easily hold the sheet extension region 81 which extends from the urine absorbing pad 10. The user can easily hold only the packaging sheet 80 without holding both the packaging sheet 80 and the urine absorbing pad 10 and can more easily perform an operation of unfolding the package and an operation of removing the urine absorbing pad 10 by one operation.

The outer end portion of the pad extension region 18 in the front-rear direction may have a folded region 19 which is folded toward the non-skin surface side in a state of being folded with the width folding line as a base point. More specifically, as illustrated in Fig. 6(b), in the second width folded state in which the folding is performed with the second width folding line FW2 as a base point, a region (a region on the other end 1Y side of the package of the first width folding line FW1 in the unfolded state) folded along the first width folding line FW1 is folded so that the urine absorbing pad 10 faces the non-skin surface side T2. Therefore, in the unfolded state, a region outside the second width folding line FW2 located closest to the other end 1Y side in the front-rear direction constitutes the folded region 19 folded toward the non-skin surface side T2 (refer to Figs. 6(a) and 6(f)). The folded region 19 of the pad extension region 18 easily stands on the non-skin surface side T2 due to the crease. Therefore, when the urine absorbing pad 10 is removed, the user can hold the folded region 19 located at the outer end portion of the pad extension region 18 in the front-rear direction L. The region held by the user can be provided at the outer end portion of the urine absorbing pad in the front-rear direction, and the entire urine absorbing pad can be more easily unfolded in the front-rear direction L.

In the one end side 1X of the urine absorbing pad package, at least a portion of the side adhesive portion 22 may be disposed outside the absorbent core 16 in the front-rear direction L. The side adhesive portion 22 may be disposed outside the outer end edge of the absorbent core in the front-rear direction, and may straddle the outer end edge of the absorbent core in the front-rear direction. The region outside the absorbent core in the front-rear direction has a lower rigidity and is more easily bent inward compared to the region where the absorbent core is disposed. Since the side adhesive portion is disposed in the region outside the absorbent core in the front-rear direction, the urine absorbing pad is hardly bent due to the rigidity of the side adhesive portion and is joined to the wearing article via the side adhesive portion. Accordingly, the urine absorbing pad is not easily bent.

In the one end side 1X of the urine absorbing pad package, at least a portion of the side sheet joining portion 76 may be disposed outside the contraction portion 13 in the front-rear direction L. In the one end side 1X of the urine absorbing pad package, the urine absorbing pad 10 can be pulled via the side release sheet 72 in a region where the contraction of the contraction portion is hardly affected. Therefore, the urine absorbing pad can be easily attached on the wearing article in a state of being unfolded to the vicinity of the side portion of the urine absorbing pad in the width direction.

In the one end side 1X of the urine absorbing pad package, at least a portion of the side sheet joining portion 76 may be disposed outside the side adhesive portion 22 in the front-rear direction. For example, in a configuration in which the side sheet joining portion 76 is located inside the side adhesive portion 22 in the front-rear direction, a point at which a force is transmitted from the side release sheet 72 to the urine absorbing pad 10 is located inside a point, at which a force is transmitted from the packaging sheet 80 to the side release sheet 72, in the front-rear direction. Therefore, in a portion in which the side adhesive portion 22 extends outward of the side sheet joining portion 76 in the front-rear direction, a force pulling the packaging sheet 80 acts only on the side release sheet 72, and there is a concern that the urine absorbing pad 10 cannot be pulled outward in the front-rear direction. However, since the side sheet joining portion 76 is located outside the side adhesive portion 22 in the front-rear direction L, when the user pulls the packaging sheet 80 outward in the front-rear direction L, a force is easily applied to the urine absorbing pad 10. Accordingly, the urine absorbing pad 10 is more easily pulled outward in the front-rear direction.

In the one end side 1X of the urine absorbing pad package, at least a portion of the side sheet joining portion may be deviated outward in the front-rear direction with respect to a center 80CL of the packaging sheet in the front-rear direction. The side sheet joining portion may straddle the center 80CL of the packaging sheet in the front-rear direction, or may be disposed only on the one end side 1X of the center 80CL of the packaging sheet in the front-rear direction. The release sheet can be pulled via the packaging sheet outside the center 80CL of the packaging sheet in the front-rear direction. In one end portion of the urine absorbing pad in the front-rear direction, the urine absorbing pad can be unfolded to be flat, and the urine absorbing pad is easily attached to the wearing article in a state where the urine absorbing pad is flatter.

As illustrated in Fig. 2, in one end side 1X of the urine absorbing pad package, a distance GL between the side adhesive portion 22 and the outer end edge of the urine absorbing pad in the front-rear direction may be shorter than a distance GW between the side adhesive portion 22 and an outer edge of the urine absorbing pad 10 in the width direction W. Since the distance between the side adhesive portion 22 and the outer end edge of the urine absorbing pad 10 in the front-rear direction is relatively short, a flat state is easily maintained to the outer end edge of the urine absorbing pad 10 during unfolding. Therefore, it is possible to suppress an inconvenience that the urine absorbing pad 10 is folded inward at the outer end edge of the urine absorbing pad 10.

A dimension of the urine absorbing pad 10 in the width direction may gradually decrease from the one end side 1X of the urine absorbing pad package toward the other end side 1Y thereof. Since a length of one end side 1X of the package 1 of the urine absorbing pad 10 in the width direction W is long, when the user holds the vicinity of the center of the urine absorbing pad 10 in the width direction W and unfolds the urine absorbing pad 10, the urine absorbing pad 10 may be difficult to be unfolded to the vicinity of the side portion of the urine absorbing pad 10 in the width direction W. However, according to the present invention, in the one end side 1X of the package 1 of the urine absorbing pad 10, the urine absorbing pad is easily unfolded to the vicinity of the side portion of the urine absorbing pad 10 in the width direction W, and even in a configuration in which a length of the one end side of the urine absorbing pad package in width direction is long, it is possible to suppress an inconvenience caused by the side portion of the urine absorbing pad in the width direction being folded inward.

At least a portion of the contraction portion 13 may be disposed outside the absorbent core 16 in the width direction W. Compared to a region where the absorbent core 16 is disposed, a region outside the absorbent core 16 in the width direction W has a lower rigidity and is difficult to be unfolded in the front-rear direction L and to be maintained in a flat state due to the contraction of the contraction portion 13. However, according to the present invention, in the one end side 1X of the package of the urine absorbing pad 10, the urine absorbing pad is easily unfolded to the vicinity of the side portion of the urine absorbing pad 10 in the width direction, and even in a configuration in which the contraction portion 13 is disposed outside the absorbent core 16 in the width direction, it is possible to suppress the inconvenience caused by the side portion of the urine absorbing pad 10 in the width direction being folded inward.

The side adhesive portion 22 may be disposed in at least one of a region overlapping the contraction portion 13 and a region extending from the contraction portion 13 in the front-rear direction L. The region overlapping the contraction portion 13 and the region extending from the contraction portion 13 in the front-rear direction L may be difficult to be unfolded in the front-rear direction L and may be difficult to be maintained in a flat state due to the contraction of the contraction portion 13. However, since the side adhesive portion 22 is disposed in at least one of the region overlapping the contraction portion 13 and the region extending from the contraction portion in the front-rear direction L, the urine absorbing pad 10 is joined to the wearing article via the side adhesive portion 22 during wearing. Accordingly, the side portion of the urine absorbing pad 10 in the width direction is hardly bent inward.

Although the embodiments of the present invention have been described above in detail, it is apparent for persons who have ordinary skill in the art that the embodiments described in this specification do not limit the scope of the present invention. Changes and modifications may apply to the embodiments of the present invention without departing from the spirit and scope of the present invention that are defined by the description of the appended claims. The description of the present specification, therefore, has been understood to be illustrative and is in no way intended to limit the scope of the invention.

The entire contents of Japanese Patent Application No. 2019-085895 (filed on April 26, 2019) have been incorporated herein by reference.

### [INDUSTRIAL APPLICABILITY]

It is possible to provide a urine absorbing pad package capable of preventing the side portion of the urine absorbing pad in the width direction from being folded inward during wearing and preventing occurrence of the leakage and the deterioration of the wearing feeling.

### [REFERENCE SIGNS LIST]

- 1: Urine absorbing pad package
- 10: Urine absorbing pad
- 11: Center sheet
- 12: Side sheet
- 13: Contraction portion
- 14: Leakage-proof gather
- 15: Backsheet
- 16: Absorbent core
- 17: Core wrap
- 18: Pad extension region
- 19: Folded region
- 21: Center adhesive portion
- 22: Side adhesive portion
- 71: Center release sheet
- 72: Side release sheet (release sheet)
- 75: Center sheet joining portion
- 76: Side sheet joining portion (sheet joining portion)
- 80: Packaging sheet
- 81: Sheet extension region
- FL1, FL2: Front-rear folding line
- FW1,: FW2 Width folding line

## Claims

1. A urine absorbing pad package (1) comprising: a urine absorbing pad (10) that has a front-rear direction (L) and a width direction (W) orthogonal to each other; a packaging sheet(80) disposed on a non-skin surface side (T2) of the urine absorbing pad; and a release sheet (70) disposed between the urine absorbing pad and the packaging sheet,
at least the urine absorbing pad being folded with a width folding line (FW1,FW2) as a base point along the width direction, the urine absorbing pad being individually packaged by the packaging sheet,
wherein the urine absorbing pad includes
an absorbent core (16),
a center sheet (11) that covers the absorbent core on a skin surface side (T1) of the absorbent core,
an upright leakage-proof gather (14) that is disposed on a skin surface side of the center sheet and has a contraction portion (13) contracted in the front-rear direction,
a backsheet (15) disposed on a non-skin surface side of the absorbent core, and an adhesive portion (21,22) that is disposed on a non-skin surface side of the backsheet and joined to a wearing article when the urine absorbing pad is used, the adhesive portion includes a center adhesive portion (21) disposed at a center of the urine absorbing pad in the width direction and a side adhesive portion (22) disposed outside of the center adhesive portion in the width direction,
the release sheet covers the side adhesive portion before the urine absorbing pad is used,
the packaging sheet is joined to the release sheet via a sheet joining portion(76),
in an unfolded state in which the urine absorbing pad is not folded with the width folding line as a base point, a sheet extension region (81) in which the packaging sheet extends outside the sheet joining portion in the front-rear direction is provided on one end side (1X) of the urine absorbing pad package in the front-rear direction,
in the unfolded state, a pad extension region (18) in which the urine absorbing pad extends outside the packaging sheet in the front-rear direction on another end side (1Y) of the urine absorbing pad package in the front-rear direction, and in the one end side of the urine absorbing pad package, at least a portion of the side adhesive portion is disposed outside the contraction portion in the front-rear direction and outside the center adhesive portion in the front-rear direction.

2. The urine absorbing pad package according to claim 1, wherein at least a portion of the sheet extension region extends outside the urine absorbing pad in the front-rear direction in the unfolded state.

3. The urine absorbing pad package according to claim 1 or 2, wherein an outer end portion of the pad extension region in the front-rear direction has a folded region (19) folded toward the non-skin surface side in a state of being folded with the width folding line as a base point.

4. The urine absorbing pad package according to any one of claims 1 to 3, wherein in the one end side of the urine absorbing pad package, at least a portion of the side adhesive portion is disposed outside the absorbent core in the front-rear direction.

5. The urine absorbing pad package according to any one of claims 1 to 4, wherein in the one end side of the urine absorbing pad package, at least a portion of the sheet joining portion is disposed outside the contraction portion in the front-rear direction.

6. The urine absorbing pad package according to claim 5, wherein in the one end side of the urine absorbing pad package, at least a portion of the sheet joining portion is disposed outside the side adhesive portion in the front-rear direction.

7. The urine absorbing pad package according to claim 5 or 6, wherein in the one end side of the urine absorbing pad package, at least a portion of the sheet joining portion is deviated outward in the front-rear direction with respect to a center of the packaging sheet in the front-rear direction.

8. The urine absorbing pad package according to any one of claims 1 to 7, wherein in the one end side of the urine absorbing pad package, a distance (GL) between the side adhesive portion and an outer end edge of the urine absorbing pad in the front-rear direction is shorter than a distance (GW) between the side adhesive portion and an outer edge of the urine absorbing pad in the width direction.

9. The urine absorbing pad package according to any one of claims 1 to 8, wherein a dimension of the urine absorbing pad in the width direction gradually decreases from the one end side (1X) of the urine absorbing pad package toward the another end side (1Y) of the urine absorbing pad package.

10. The urine absorbing pad package according to any one of claims 1 to 9, wherein at least a portion of the contraction portion is disposed outside the absorbent core in the width direction.

11. The urine absorbing pad package according to any one of claims 1 to 10, wherein the side adhesive portion is disposed in at least one of a region overlapping the contraction portion and a region extending from the contraction portion in the front-rear direction.
